# EUROPEAN PATENT APPLICATION

(11) **EP 3 795 184 A1**
(43) Date of publication of application: **24.03.2021**
(21) Application number: 19198711.4
(22) Date of filing: 20.09.2019
(51) Int. Cl.: A61L 27/22, A61L 27/24, A61L 27/38, A61L 27/56, A61L 27/60

(54) **CELLULAR SUBSTITUTES AND METHODS OF PREPARATION THEREOF**

(71) Applicant: Les Hôpitaux Universitaires de Genève, 1205 Geneva (CH); UNIVERSITE DE GENEVE, 1211 Geneva 4 (CH)
(72) Inventor: BREMBILLA, Nicolò Costantino, 1212 Grand-Lancy (CH); PREYNAT-SEAUVE, Olivier, 74420 Habère-Lullin (FR); BOEHNCKE, Wolf-Henning, 74100 Etrembières (FR); KRAUSE, Karl-Heinz, 1206 Genève (CH)
(74) Representative: reuteler & cie SA

(57) **Abstract**

The present invention is directed to tissue substitute materials and uses thereof useful for the treatment of wound, skin wounds and in particular chronic ulcers. The invention further relates to methods of preparation of those tissue substitute materials.

## Description

### Field of the Invention

The present invention relates to cellular substitutes, in particular skin substitutes and uses thereof for the treatment of wounds and in particular chronic ulcers. The invention further relates to methods of preparation of those substitutes.

### Background of the Invention

Chronic ulcers (CUs) are defined as a disruption of tissues, frequently the skin, that does not heal spontaneously or take more than six weeks to heal. CUs are the result of a complex cascade of pathogenetic factors and are associated with a multitude of diverse medical conditions, including diabetes, vascular dysfunction, infections, and immobility/pressure (use of wheel chair or long stay in bed). An important common denominator of CUs is the presence of tissue ischemia. CUs affect more than 1% of the general population and up to 4% of people over the age of 80 years. About 10% of patients affected by diabetes presents CUs in the form of Diabetic Foot Ulcers (DFUs). These latter are chronic pedal wounds associated with infection, pain, skin discoloration, and occasional bleeding. This is due to accumulating vessels injuries, which render the tissues more prone to damage by minor pressure. DFUs, and CU in general, are extremely painful and determine an important reduction of the patient quality of life, often culminating in depressive disorders. CUs expose to the risk of chronic infection and amputation. Limiting to the cases of DFUs, about 66% of patient must undergo amputation, representing more than 1000 cases per year in Switzerland (*Revue Médicale Suisse*-2440/23066). CUs result from a combined angiopathy and neuropathy, causing reduced sensation in the affected tissue (Tahergorabi and Khazaei, 2012, Int J Prev Med, 3(12), 827-38). An unnoticed trauma in the affected area without sufficient blood flow to ensure wound healing then provides an optimal milieu for injury and infection, resulting in chronic skin ulceration. In addition, CUs may originate as a result of skin burns, trauma, prolonged physical pressure or surgery. The current treatments of CUs have a limited efficacy, including wound dressing, local use of growth factors, application of animal dermo-epidermic substitutes, exudate removal, hyperbaric oxygen therapy (Kessler, et al., 2003, Diabetes Care, 26(8), 2378-82) and debridement of necrotic tissues. Because of their xenogenic origin, the used porcine dermo-epidermic substitutes (Apligraf®, Organogenesis Inc., USA) provide transitory inflammatory-like reactions helping for healing, but rapidly rejected. In light of these facts, a large number of studies focus on novel biological therapies that may be used to treat CUs.

Adipose-derived Stem Cells (ASC) are a component of the human adipose tissue producing large amounts of most of the factors needed for an efficient wound healing. Autologous ASCs are easily isolated and expanded in culture through lipo-aspiration or fat sampling. ASCs are classified with specific criteria from the International Federation of Adipose Therapeutics and Science (IFATS) (Bourin et al., 2013, Cytotherapy, 15(6), 641-8) and are currently one of the most studied cells for regenerative cell therapy applications (Si, et al., 2019, Biomed. Pharmacother., 114: 108765*).*

ASCs are considered as being an encouraging treatment option for chronic wounds since numerous preclinical studies in animals demonstrated that ASCs transplanted in the ulcer environment favor wound repair (Gdelkarim et al., 2018, Biomed. Pharmacother., 107, 625-633) through collagen/matrix secretion and deposition, growth factor secretion, angiogenesis and re-epithelization. Even if multiple intramuscular injection of ASC in liquid suspension might be a favorable therapeutic option in patients with DFU (Lee et al., 2012, Circ. J., 76(7), 1750-60*;* Bura et al., 2014, Cytotherapy, 16(2), 245-57), their physical/temporal stability within the wound bed remains a critical issue to address. Indeed, it has been reported that injected ASC are locally unstable, display high motility and ultimately spread to sites far from the wound (Zhao et al., 2016, Cytotherapy, 18(7) 816-27).

Thus, since CUs are a major cause of morbidity and mortality with increasing prevalence, there is a need to find novel treatments and skin substitute solutions. In particular, in view of the promising nature of ASC-based therapies, there is a need for new tools that would allow solving the problems limiting their development as an efficient treatment in regenerative medicine.

### Summary of the invention

The present invention relates to the unexpected finding of a method of preparation of a skin substitute material which behaves like a trophic auto-graft and is clinical-grade compatible, bioresorbable and not rejected by the patient. These findings provide a promising cost-effective approach for skin regenerative medicine which has been validated *in vitro* by using 4 different ASC lines from patients with chronic ischemia, including one presenting diabetic foot ulcers (DFU).

An object of this invention is to provide a tissue substitute material useful for tissue regeneration or biological tissue culture (*in vitro* or *ex-vivo*) and tissue engineering or cosmetic applications.

It is useful to provide a tissue substitute material which actively produces high amounts of wound healing factors.

It is useful to provide a tissue substitute material which allows concentrating locally adipose-derived stem cells and their secreted factors.

It is useful to provide a tissue substitute material containing the cells of a subject (i.e. autologous) to avoid tissue graft rejection.

It is useful to provide a tissue substitute material with trophic/regenerative activity to heal wounds and allows protecting the wound by skin-engraftment.

It is useful to provide a tissue substitute material generated from a matrix which is bioresorbable.

It is useful to provide a tissue substitute material which allows treating both superficial and deep wounds.

It is useful to provide a tissue substitute material which reduces the time spent by healthcare personnel for wound care and the associated costs.

Objects of this invention have been achieved by providing a tissue substitute material according to claim 8 or 12 and materials according to claim 14 derived therefrom, a method for obtaining a tissue substitute material according to claim 1 and uses thereof.

Disclosed herein, according to a first aspect of the invention, is a method for the preparation of a tissue substitute material, said method comprising the following steps:
a) Providing Adipose-derived Stem Cells (ASCs);
b) Loading the said ASCs into a scaffold sponge (e.g. Gelfoam);
c) Placing the obtained loaded sponge onto to a hemi-permeable membrane floating over an ASC culture medium;
d) Incubating under ASC culture conditions the obtained floating loaded sponge to let the ASC growing and expanding within the said floating sponge;
e) Obtaining an ASC-containing sponge suitable as a tissue substitute material or as a substrate for the preparation of a tissue substitute material.

According to another aspect of the invention, is provided a tissue substitute material comprising a scaffold sponge (e.g. Gelfoam) comprising Adipose-derived Stem Cells (ASCs) expanded within said scaffold sponge under air-liquid interface conditions.

According to another particular embodiment, is provided a reconstruction tissue comprising a tissue substitute material according to the invention.

Another aspect of the invention relates to a wound dressing comprising a tissue substitute material according to the invention.

Another aspect of the invention relates to a cosmetic composition comprising at least one tissue substitute material according to the invention.

Another aspect of the invention relates to a soft tissue filler comprising at least one tissue substitute material or a composition thereof according to the invention.

Another aspect of the invention relates to an *in vitro* or *ex vivo* cell or biological tissue culture substrate or medium comprising tissue substitute material or a composition thereof according to the invention.

Another aspect of the invention relates to a tissue substitute material according to the invention for use in tissue regeneration or in the treatment of tissue wounds, in particular chronic ulcers.

Another aspect of the invention relates to a tissue substitute material according to the invention for use in tissue engineering or in cosmetic applications.

Another aspect of the invention relates to a use of a tissue substitute material according to the invention for the preparation of a wound dressing or a reconstruction tissue for the treatment of internal organ and skin wounds, in particular chronic ulcers.

Another aspect of the invention relates to a method of treating or ameliorating tissue injuries, said method comprising contacting the tissue of said subject in need thereof with a tissue substitute material according to the invention.

Another aspect of the invention relates to a kit for wound dressing comprising at least one tissue substitute material of the invention.

Another aspect of the invention relates to a kit for the preparation of a tissue substitute material of the invention or part of it.

### Description of the figures

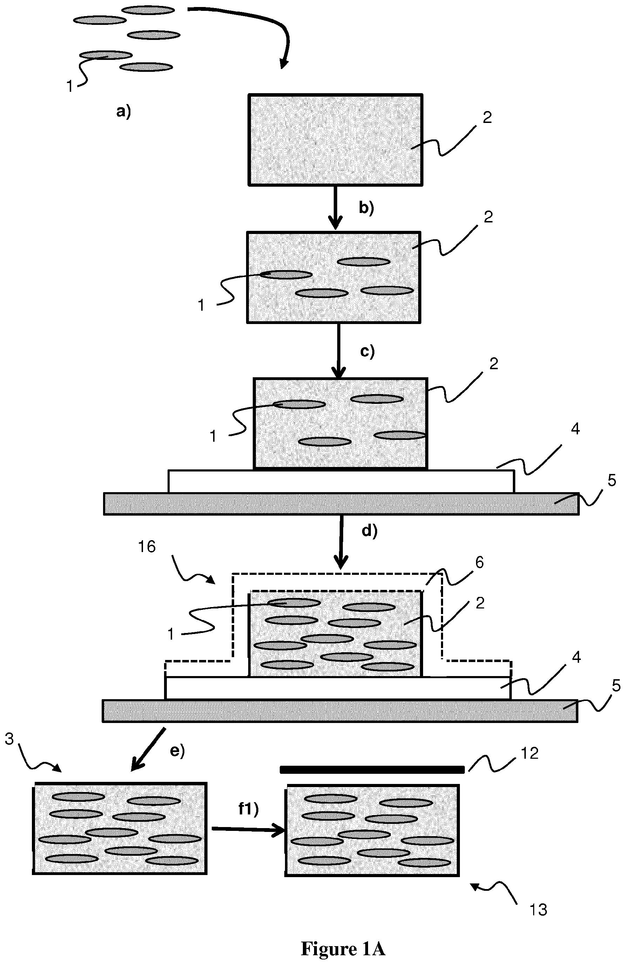

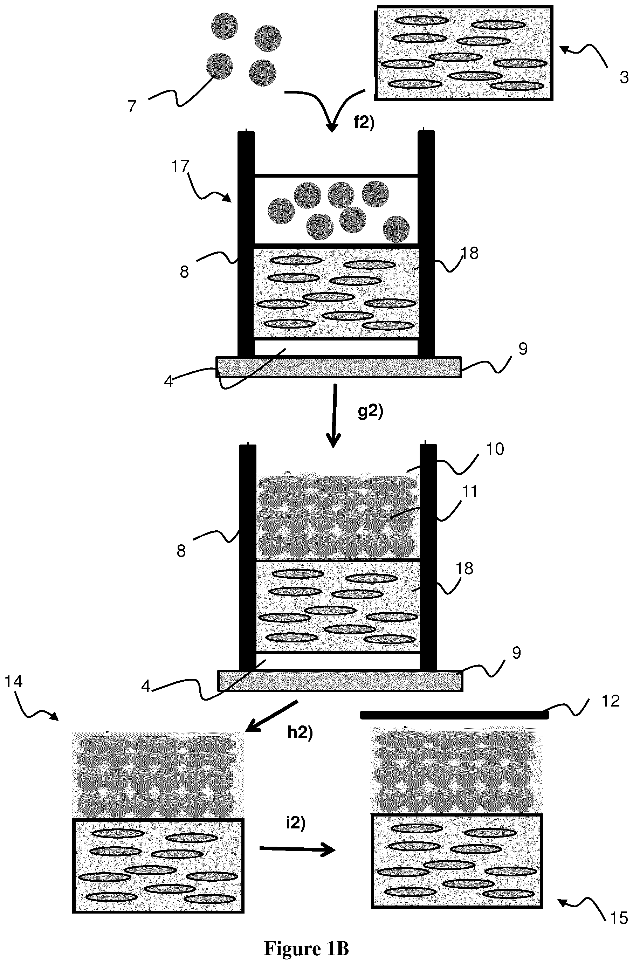

**Figure 1** is a schematic representation of the main steps of a method of the invention for obtaining a tissue substitute material of the invention which can be used as such or covered with a protective membrane or used as a substrate for the preparation of a tissue substitute material comprising a fully stratified epidermis said method comprising a scaffold sponge **2** comprising ASCs **1** expanded within said sponge under air-liquid interface conditions where a hemi-permeable membrane **4** floating on culture medium **5,** defining air/liquid interface conditions **6,** thereby leading to a tissue substitute material of the invention in the form of an ASC/sponge tissue substitute material as described in Example 1 which can be used as such **3** or as further covered **13** by a protective membrane **12** (**Fig. 1A**). The obtained ASC/sponge tissue substitute material **3** can be used as a substrate for the preparation of a tissue substituted material comprising a fully stratified epidermis (**Fig. 1B**) where the obtained ASC/sponge tissue substitute material **3** is placed in a top chamber **18** of a cell culture insert **8** containing a hemipermeable membrane **4** at the bottom, said hemipermeable membrane of said cell culture insert **8** floating over a culture medium **9,** and the ASC/sponge tissue substitute is further covered with keratinocytes **7** in a keratinocyte culture medium, thereby defining air interface conditions (**10**) over the keratinocyte layer, allowing the formation of a fully stratified epidermis **11** on top of the ASC/sponge tissue substitute, hereby leading to a tissue substitute material of the invention in the form of an ASC/sponge/epidermis tissue substitute composite as described in Example 4 which can be used as such **14** or further covered **15** with a protective membrane **12.**
**Figure 2** shows the multipotent properties of ASC used in Example 1. **A:** ASCI-derived adipocytes characterized by the adipocyte marker FABP4 **B:** ASCI-derived osteoblasts characterized by the osteoblast marker osteocalcin. **C:** ASCI-derived chondrocytes characterized by the chondrocyte marker aggrecan.
**Figure 3** shows the expansion of the ASC1 in the scaffold sponge before (Day 0) or after one-week (Day 8) culture in paraffin-imbedded sections colored with hemalun/eosin as described in Example 1.
**Figure 4** shows the regulation of ASC positive or negative markers (mRNA) in the culture conditions of the skin substitute material of the invention as described in Example 2 for four different ASC lines after one week of culture.
**Figure 5** shows the results of a principal component analysis (TAC4.0 software from Applied biosystems) of transcriptomes from three different ASC lines cultured in 2D or gelatin sponge conditions as described in Example 2. Human Foreskin Fibroblasts (HFF) were used as a "regenerative "control whereas Embryoid Bodies (EB) derived from the HS420 embryonic stem cell line as a "non-regenerative control".
**Figure 6** characterizes the regulation of regenerative factors (mRNA) under sponge culture conditions as described in Example 2 for three different ASC lines from different patients after one week and expressed as a fold change between sponge and 2D condition for 75 genes involved in wound healing and found to be positively expressed in the transcriptome analysis.
Only significant changes (according p value, p<0.05) are presented.
**Figure 7** quantifies some regenerative factors in the supernatants of sponge versus 2D culture conditions as measured in by a Luminex bioarray technology as described in Example 2 for four different ASC lines from different patients after one week.
**Figure 8** quantifies some matrix metalloproteases in the supernatants of sponge versus 2D culture conditions as measured in by a Luminex bioarray technology as described in Example 2 for four different ASC lines from different patients after one week.
**Figure 9** shows a principal component analysis of transcriptomes from ASC 10 at three different times of culture as described in Example 3.
**Figure 10** represents the levels of sternness markers (Oct-4, Nanog) and tumorigenicity marker hTERT (A) and FGF-7 (**B**) in a skin substitute material of the invention measured as described in Example 3.
**Figure 11** shows histological coloration of a skin substitute material protected by a reconstructed epidermis as described in Example 4 (top panels: H&E staining and pan-keratinocyte immunostaining.
**Figure 12** compares H&E stainings of a skin substitute material protected by a reconstructed epidermis after 20 days of culture (right) as described in Example 4 with a normal epidermis (left) as described under Example 4. Epidermal strati are indicated: **1:** *Stratum basale*; **2:** *Stratum spinosum*; **3:** *Stratum granulosum;* **4:** *Stratum corneum.*
**Figure 13** compares the levels of regenerative factors after addition of an epidermis-like layer on a skin substitute material of the invention to a non-protected skin substitute material of the invention as analyzed by micro-array (Affymetrix) as described in Example 5 and expressed as the fold change expression of the 75 mRNA of regenerative factors between the ASC/sponge condition and the ASC/sponge/keratinocytes condition.
**Figure 14** shows significant regulations (mRNA) of regenerative factors between ASC/sponge/keratinocytes (protected skin substitute material of the invention) and (non-protected skin substitute material of the invention) ASC/sponge conditions expressed in fold change expression. Genes were classified according a statistically significant fold change (p<0.05) between the two conditions

### Detailed description

The term "pharmaceutically acceptable" refers to a carrier comprised of a material that is not biologically or otherwise undesirable and not especially toxic.

The term "tissue substitute" or "cellular substitute" relates to a substitute material which may replace native tissue or cells from a subject that have been damaged. In a particular embodiment, tissue or cellular substitutes according to the invention include skin substitutes and visceral tissue substitutes.

The term "scaffold sponge" is used herein to describe a gelatin or collagen or collagen-gelatin sponge such as sterile collagen sponges used as a topical hemostatic device in surgery (such as Gelfoam™ Pfizer, New York NY, SURGIFOAM® from J&J, SPONGEL from EthanMedical, CUTANPLAST® from Kms Group, REKO GELATAMP from Coltène, SPONGOSTAN from Ferrosan medical devices, CURASPON from curamedical, CUTANPLAST from dentaltix, ATELOCELL from cosmobio, or AVITEN ULTRAFOAM from Becton Dickinson/BARD, CODMAN/BCOL from Integra). According to a particular aspect, a scaffold sponge according to the invention, when being a gelatin sponge may be prepared as by standard techniques described in Kabiri et al., 2011, Current Applied Physics, 11(3), 457-461*;* Rafienia et al., 2008, Iranian Journal of Pharmaceutical Sciences Summer, 4(3): 193-200*; Peer, 2017, 9;5:e3665;* Imani et al., 2013, Biomed Mater Eng. 23(3):211-24. doi: 10.3233/BME-130745*.*

According to one aspect of the invention, a scaffold sponge suitable for the invention is bioresorbable within about one month in soft tissues.

According to another aspect of the invention, a scaffold sponge suitable for the invention is easily cuttable for adapting to the size of the wounds.

According to another aspect of the invention, a scaffold may be generated by 3D printing of biologic or synthetic material.

The term "ASC culture medium" comprises a suitable culture medium for Adipose-derived Stem Cells such as described for example in Riis et al., 2016, Stem Cells Transl Med., 5(3): 314-324 or commercially available media under the following names: Gibco StemPro MSC SFM, Mesenpro RS medium, StemPro MSC SFM XenoFree media (all from Gibco), Peprogrow medium form Peprotech, Mesenchymal stem cell growth medium DXF from Promocell, Mesenchymal stem cell medium from Sciencell, MesenCult MSC Basal Medium from StemCell Technologies, StemXVivo® Serum-Free MSC Expansion Media from R&D, MSCBMTM Basal Media from Lonza or as described in *Uckay et al., 2019, supra.*

The term "protective membrane" is used herein to cover any suitable layer that would allow to protect the skin substitute material from bacterial infection and which would be vapour permeable membrane such as polyurethane film as described in Xu et al., Scientific Reports 2016 (commercially available products include Tegaderm from 3M and Opsite from Smith & Nephew) or a epidermis layer derived from a keratonocyte layer for example prepared as described herein.

The term "skin wounds" includes skin damages where the skin surface presents sore depression without necessarily a cut on its surface such as age-related tissue damages (e.g. wrinkles), wounds and scars such as for example acne or rubella scars. Those disorders further include wounds, scars, acne. The term "wounds", includes any damaged tissue, for example following trauma or surgery. Wounds in mammals include for examples abrasions lacerations, contusions, stab wounds, skin cuts, surgical wounds, gunshot wounds, thermal wounds, chemical wounds, bites and stings and electrical wounds. It further includes chronic skin disorders such as skin ulcers (e.g. chronic ulcers of various etiology: venous, ischemic, diabetic, pressure).

The term "reconstruction tissue" refers to a biological tissue (endogeneous or exogeneous) or a synthetic or semi-synthetic material useful for repairing damaged tissues of the body (e.g. skin reconstruction tissue, visceral reconstruction tissue, vascular reconstruction tissue etc.). As used herein, "treatment" and "treating" and the like generally mean obtaining a desired pharmacological and/or physical and/or physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term " treatment" as used herein covers any treatment of a disease in a mammal, particularly a human, and includes inhibiting the disease, i.e., arresting its development; or relieving the disease, i.e., causing regression of the disease and/or its symptoms or conditions such as improvement or remediation of damage.

The term "subject" as used herein refers to mammals. For examples, mammals contemplated by the present invention include human, primates, and domesticated animals such as cattle, sheep, pigs, horses and particularly race horses, laboratory rodents and the like.

The term "efficacy" of a treatment according to the invention can be measured based on changes in the course of disease in response to a use according to the invention. For example, the efficacy of a treatment according to the invention can be measured by a reduction of the surface and/or depth of the wound, or by a faster time for complete healing, or a reduction in the associated-pain, or a reduced infection rate.

### Method of preparation of a tissue substitute material according to the invention

Referring to the figures, in particular first to Figures 1A and 1B, is provided an illustration of a method for preparation of a tissue substitute material comprising the steps of:
a) Providing Adipose-derived Stem Cells (ASCs) (**1**);
b) Loading the said ASCs into a scaffold sponge (**2**);
c) Placing the obtained loaded scaffold sponge onto to a hemi-permeable membrane (**4**) floating over an ASC culture medium (**5**);
d) Incubating under ASC culture conditions the obtained floating loaded scaffold sponge (16) to let the ASC growing and expanding within the said floating scaffold sponge;
e) Removing the cultured floating loaded scaffold sponge from the hemi-permeable membrane (**4**) and obtaining an ASC-containing sponge suitable as a tissue substitute material (**3**).

According to a further particular aspect, the method according to the invention may further comprise a step f1) wherein the obtained ASC-containing sponge (**3**) is covered by a protective membrane (**12**) to obtain a protected tissue substitute material (**13**). This type of substitute material will be particularly suited for the treatment of superficial or deep wounds, including both wounds in the skin and other tissues.

According to a further particular aspect, the method according to the invention may further comprise a step f2) wherein the ASC-containing sponge obtained under step e) is further coated with keratinocytes in keratinocyte culture medium to allow the formation of a stratified epidermis over the surface of the covered ASC-containing sponge.

According to another further particular aspect, the method according to the invention may further comprise the following steps:
f2) placing the obtained ASC-containing sponge (**3**) in a top chamber (**18**) of a cell culture insert (**8**) containing a hemipermeable membrane (**4**) at the bottom, said cell culture insert (**8**) floating on the surface of a culture medium (**9**) and covering the ASC-containing sponge (**3**) with keratinocytes (**7**) (e.g. autologous) in keratinocyte culture medium to form a keratinocyte-covered ASC-containing sponge (**17**) within the said cell culture insert;
g2) incubating the keratinocyte-covered ASC-containing sponge (**17**) under keratinocyte culture conditions thereby defining air interface conditions (**10**) over the keratinocyte layer and allowing a stratified epidermis (**11**) to grow on the surface of ASC-containing sponge under air conditions;
h2) removing the ASC-containing sponge from top chamber (**18**) of the cell culture insert (**8**) and obtaining an ASC-containing sponge covered with a stratified epidermis as a tissue substitute material (**14**). The stratified epidermis provides a protective layer and this type of substitute material will be particularly suited for the treatment of superficial wound or in reconstructive-based approach for the treatment of skin wound as defined above.

According to a particular embodiment, the incubation medium is replaced after about 24 of incubation under step g2), at least once, by a keratinocyte differentiation medium, and removed to further create air interface conditions (**10**) and the formation of the stratified epidermis (11) on its keratinocyte-covered surface.

According to another further particular aspect, the method according to the invention comprises a further step wherein the ASC-containing sponge obtained in the last step is further coated with a protective membrane such as a vapour-permeable membrane (e.g. a polyurethane film).

According to another further particular embodiment, is provided a method for preparation of a tissue substitute material wherein the ASC-containing sponge obtained under step e) is further coated with a protective membrane such as for example a vapor permeable membrane such as polyurethane film as described in Xu et al., 2016, Scientific Reports*, supra* or an epidermis layer derived from a keratinocyte layer, for example prepared as described herein.

According to another further particular aspect, the method according to the invention may further comprise a step i2) wherein the obtained ASC-containing sponge covered with a stratified epidermis (**14**) is covered by a protective membrane (**12**) to obtain a protected tissue substitute material (**15**).

According to another particular embodiment, the scaffold sponge is loaded with about 100'000 to about 200'000 Adipose-derived Stem Cells (autologous or non autologous).

According to another particular embodiment, the hemi-permeable membrane can be made of polytetrafluoroethylene, polycarbonate, polyethylene terephthalate or cellulose esters.

According to another particular embodiment, is provided a method for preparation of a tissue substitute material wherein the floating loaded sponge is incubated under step d) for from about 5 to 20 days under ASC culture conditions.

According to another particular embodiment, is provided a method for the preparation of a tissue substitute material wherein the keratinocyte-coated ASC-containing sponge is incubated under step g2) for from about 10 to about 15 days in the top chamber of the cell culture insert containing at the bottom a hemipermeable membrane floating on a keratinocyte culture medium.

### Tissue substitute material according to the invention

According to a particular embodiment, is provided a tissue substitute material obtainable from a method according to the invention.
According to a particular embodiment, is provided a tissue substitute material comprising a scaffold sponge comprising Adipose-derived Stem Cells (ASCs) expanded within said sponge under air-liquid interface conditions.
According to a particular embodiment, the scaffold sponge is a gelatin sponge (e.g. Gelfoam). According to a particular embodiment, air-water conditions comprise a hemi-permeable membrane as a support for the ASC-containing scaffold sponge floating on an ASC culture medium.
According to another particular embodiment, a tissue substitute material according to the invention comprises about 150'000 to about 1'500'000 Adipose-derived Stem Cells (autologous or non autologous).
According to a particular embodiment, is provided a tissue substitute material which can be frozen, transported and used at the bedside.
According to a particular embodiment, is provided a tissue substitute material which can be packaged before use.
According to a particular embodiment, is provided a tissue substitute material which is suitable for internal wounds, in particular which is suturable.
According to a particular embodiment, the size of a tissue substitute material can be adjusted to the size of the wound to treat, e.g. but cutting the sponge.

According to a further particular embodiment, a tissue substitute material of the invention has a size ranging from about 1 to about 30 cm².

According to another particular embodiment, is provided a tissue substitute material of the invention has a thickness ranging from about 1 mm to about 1 cm.
According to a particular embodiment, is provided a tissue substitute material according to the invention comprising a protective membrane such as keratinocyte layer or a vapour-protective membrane (e.g. a polyurethane film).
According to a particular embodiment, a tissue substitute material of the invention comprises a protective membrane on one side of the scaffold sponge.
According to a particular embodiment, a tissue substitute material of the invention comprises a keratinocyte layer membrane on one side of the scaffold sponge.
According to a particular embodiment, is provided a wound dressing, a reconstruction tissue, a cosmetic composition or a soft tissue filler comprising a tissue substitute material of the invention.
According to a particular embodiment, a wound dressing, a reconstruction tissue, a cosmetic composition or a soft tissue filler of the invention may further comprises at least one anti-scar agent, or anti-aging agent or injectable filler such as collagen, hyaluronic acid, calcium hydroxylapatite, poly-1-lactic acid and polymethylmethacrylate.
According to a further particular embodiment, for cosmetic applications, fat, fat substitutes or fillers can be further introduced within the sponge by simple impregnation before the loading of the ASC.
Another aspect of the invention relates to a kit for wound dressing comprising at least one tissue substitute material of the invention.
Another aspect of the invention relates to a kit for the preparation of a tissue substitute material, containing a scaffold sponge and at least one further element of the following group: keratinocytes in keratinocyte culture media, ASC culture media, a cell culture insert containing a hemipermeable membrane, cell culture plates and frozen cells.
According to a particular embodiment, a tissue substitute material according to the invention is a skin substitute.
According to another particular embodiment, a tissue substitute material according to the invention is a visceral tissue substitute.
According to a particular embodiment, tissue substitute materials of the invention are suitable for topical use.
Alternatively the invention provides material that can be used in another mammal thus humans for the same uses as described above.

The invention further provides material or methods useful for reconstruction surgery (e.g. tissue reconstruction), in tissue regeneration or tissue engineering or for the treatment of internal organ and skin wounds, in particular chronic ulcers or in cell or biological tissue culture.

### Mode of administration

According to a particular embodiment, the tissue substitute material of the invention is removed from the hemi-permeable membrane and placed directly over internal organ wound (as defined below).

According to a particular embodiment, the tissue substitute material of the invention is removed from the hemi-permeable membrane and placed on a wound (as defined below) with a protective member (e.g. polyurethane) on the side facing the environment external to the damaged tissue.

According to a particular embodiment, the tissue substitute material of the invention is removed from cell culture insert and placed directly over internal organ wound (as defined below).

According to a particular embodiment, the tissue substitute material of the invention is removed from cell culture insert and placed on a wound (as defined below) with a protective membrane (e.g. polyurethane) on the side facing the environment external to the damaged tissue.

Materials of this invention may be applied to damaged external tissue (e.g. skin) in any manner by direct application of the tissue substitute material, the non-protected side onto the damaged tissue. Materials of this invention may be applied to damaged internal tissue (e.g. visceral tissue) in any manner by direct application of the tissue substitute material in the absence of protective layer. Materials of this invention may be applied side by side or one above the other for the treatment of large or deep wounds, respectively.

### Combination

According to one aspect, tissue substitute materials of the invention may be applied alone or in combination with at least one co-agent useful for tissue regeneration.

According to a particular aspect, co-agents according to the invention include growth factors, platelet gels, platelet-rich plasma, platelet concentrates, injectable fillers, other cells such as stem cells, vascular progenitors derived from stem cells, stromal cells, stromal vascular fraction.

According to one aspect, skin substitute materials of the invention may be applied alone or in combination with at least one antimicrobial agent.

According to a particular aspect, co-agents according to the invention include antibiotics, metal or metal-based nanoparticles such as neomycin and silver particles.

### Patients

In an embodiment, patients according to the invention are subjects suffering from tissue degeneration, skin injuries or wounds.

In a further embodiment, patients according to the invention are subjects suffering from skin wounds, in particular skin ulcers.

In another further embodiment, patients according to the invention are subjects suffering from internal tissue injuries, in particular visceral ulcers.

### Use according to the invention

According to a particular embodiment, the tissue substitute materials of the invention of the invention and compositions thereof are useful for various applications such as *in vitro* cell or biological tissue culture, as tissue fillers, for cosmetic, and tissue engineering applications. According to another particular embodiment, is provided a use of tissue substitute material according to the invention for the preparation of a wound dressing or of tissue engineering materials.

According to another particular embodiment, the skin substitute materials of the invention and compositions thereof are useful for use in the treatment of a medical disorder, and in particular skin wounds or injuries.

Examples illustrating the invention will be described hereinafter in a more detailed manner and by reference to the embodiments represented in the Figures.

### EXAMPLES

The following abbreviations refer respectively to the definitions below:
**DAPI** (4',6-Diamidino-2-Phenylindole, Dihydrochloride), **DMEM** (Dulbecco's Modified Eagle Medium), **EDTA** (Ethylenediaminetetraacetic acid), **HBSS** (Hank's Balanced Salt Solution), **PFA** (Paraformaldehyde)

### Example 1: Preparation of a tissue substitute material of the invention

A method for the preparation of a tissue substitute material according to the invention illustrated on **Figure 1A** and **1B** was carried out.

In particular, the method comprised the following steps:

### a) Providing ASCs

ASC **(1)** were isolated from fat tissue taken from abdominal or skin samples of various chronic ischemic patients, including one with diabetic foot ulcer as detailed in Table 1. The protocol used was compatible with a clinical use in humans, excluded non-chemically defined and animal compounds.

**Table 1**

| **ASC line name** | **#1** | **#2** | **#3** | **#4** |
|---|---|---|---|---|
| **age** | 50 | 64 | 49 | 72 |
| **gender** | F | M | M | M |
| **ischemia** | leg | leg | foot | foot |
| **disease** | radiodermitis synoviosarcoma | malformation gut cancer alcool | trauma amputation | diabetes chronic ulcer amputation |
| **chronic ulcer** | no | no | no | yes |
| **diabetic** | no | no | no | yes |
| **smoker** | no | no | no | no |
| **derivation** | 2018 02 21 | 2018 02 26 | 2018 03 13 | 2018 08 13 |
| **fat collection** | leg | foot | foot | foot |
| **phenotype** | yes | yes | yes | yes |
| **Differentiation** adipocytes chondrocytes osteocytes | yes | yes | yes | yes |

To generate the ASC lines, 50 ml of fragmented fat tissue was used for the procedure. Fat was separated from the skin with scalpels, fragmented in small pieces of 2 mm length, and immediately immersed into HBSS medium (1:1 volume ratio) containing calcium and magnesium (Thermofisher) and penicillin-streptomycin 1 U/ml (Thermofisher). Fat can also be already fragmented in small pieces by the standard lipo-aspiration procedure such as described in Kølle SF et al., 2013, Lancet.,382(9898):1113-20*.* After centrifugation for 5 min at 300 x g, fat fragments were washed three times with HBSS medium and maintained under constant agitation at 37°C for 90 min in the presence of collagenase NB4 0.6 U/ml (Amsbio). Digested suspension was then diluted with an equal volume of HBSS medium and filtered through cell strainers (100 µm) to remove residual fragments. Following centrifugation at 300 x g for 5 min, the pellet was re-suspended in 50 ml of HBSS medium, and the suspension filtered on a cell strainer (100 µm). The procedure was repeated three times and the cells were seeded on tissue culture flasks at a density of 85 000 cells/cm² in complete ASC culture media (DMEM 4.5 g/l glucose with Glutamax (Thermofisher), 10% human platelet lysate (Macopharma), penicillin/streptomycin 1 U/ml (Thermofisher)) after being filtered through cell strainer (40 µm). After 24 hours of culture at 37°C/5% CO₂, the medium was changed every 3 days. At one week, cells were passaged by enzymatic dissociation using 0.5% trypsin/0.2% EDTA (Thermofisher) (for a maximum of ten times).

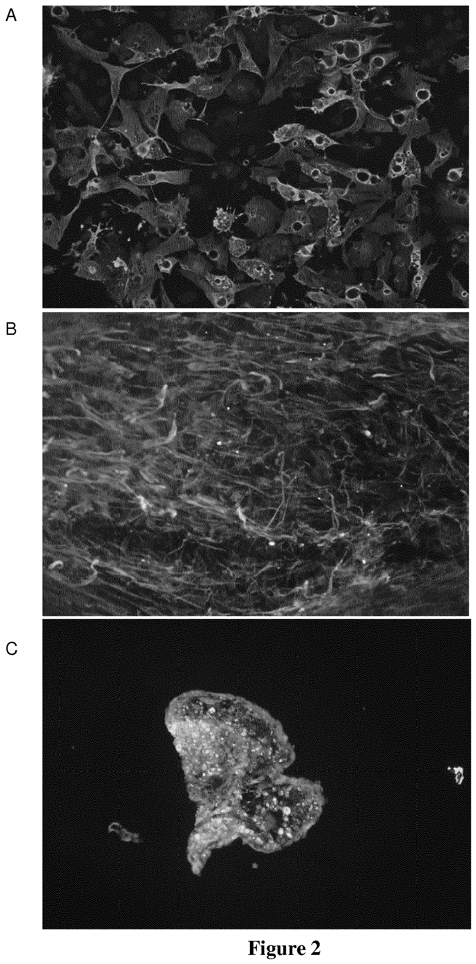

The four ASC lines derived from those patients show that all expressed the expected surface markers with positivity for CD73, CD105, CD90 and negativity for CD45, HLA-DR and CD34 (Table 1).. In the presence of appropriated media designed for *in vitro* multipotent differentiation of ASC (MSC functional identification kit, R&D Systems), all ASC differentiated towards: **i)** adipocytes expressing the specific marker fatty acid binding protein 4 (FABP4) (**Figure 2A**); **ii)** osteocytes expressing osteocalcin (**Figure 2B**); **iii)** chondrocytes expressing aggrecan (**Figure 2C**).

### b) Loading the said ASCs into a gelatin scaffold sponge

Each ASC line was introduced within a surgical sponge made of porcine gelatin (Gelfoam®, Pfizer) **(2)** at a density of 150 000 ASCs/mm³ by mechanical impregnation of the sponge with a cell suspension in the same ASC culture medium as defined previously (300 µl of cell suspension for 200 mm³ of sponge).

### c) Placing the obtained loaded sponge onto to a hemi-permeable membrane floating over an ASC culture medium

The obtained loaded sponge was then deposited on a hemi-permeable membrane of polycarbonate (Millicell Cell culture insert, 30 mm, polycarbonate, 0.4 µm, Millipore).
**(4)** floating over 1ml ASC culture medium **(5).**

### d) Incubating under ASC culture conditions the obtained floating loaded sponge to let the ASC growing and expanding within the said floating sponge

The culture medium was changed every 3 days. The air-liquid culture condition used allows a better exchange of gas with the air due to the formation of a very thin film of medium on the sponge **(6)** (**Figure 1A**). This culture condition is essential for an efficient culture of ASC within the gelatin sponge.

### e) Obtaining an ASC-containing sponge suitable as a tissue substitute material

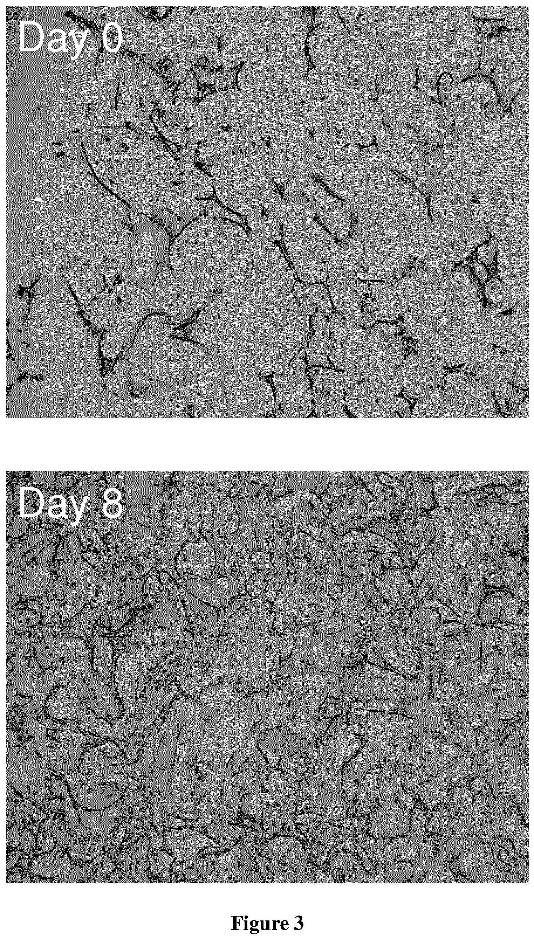

As can be seen on **Figure 3**, after one week, the ASCs efficiently grew within the sponge as showed by a higher number of cells and gelatin fibers retraction confirming their ability to survive and expand within a stable/biocompatible/bioresorbable material. The ASC were physically stable for at least five weeks within the sponge, and kept an ASC-phenotype. This was assessed by flow cytometry and multipotent differentiation after subculture of the cells extracted from the sponge in standard culture conditions.

Those data support that under the air-liquid culture condition of the method of the invention, the obtained ASC-containing sponge was a stable and compact tissue-like composite.

### Example 2: Functional characterization of the ASC/sponge composite and comparison with standard monolayer cultures

A full transcriptome analysis by microarray was performed as described below to estimate the regenerative properties of the ASC/sponge composite material obtained according to a method of the invention. ASC from 3 different donors (ASC2, ASC3, ASC4) were cultured for 8 days either in the sponge (ASC/sponge) according to protocol described in Example 1 or in standard 2D monolayer conditions on plastic tissue culture flasks (ASC/2D).

### Microarrays

Microarrays targeting 21 448 mRNA chips were performed on the extracted RNA by Complete GeneChip® Instrument System, Affymetrix® (www.affymetrix.com). The heatmap and fold change analysis were performed on RMA normalized data produced with the software TAC 4.0.1.36 (applied Biosystems®) using the R package pheatmap (https://cran.r-project.org/web/packages/pheatmap/index.html) default parameters. No transformation was applied to the data.

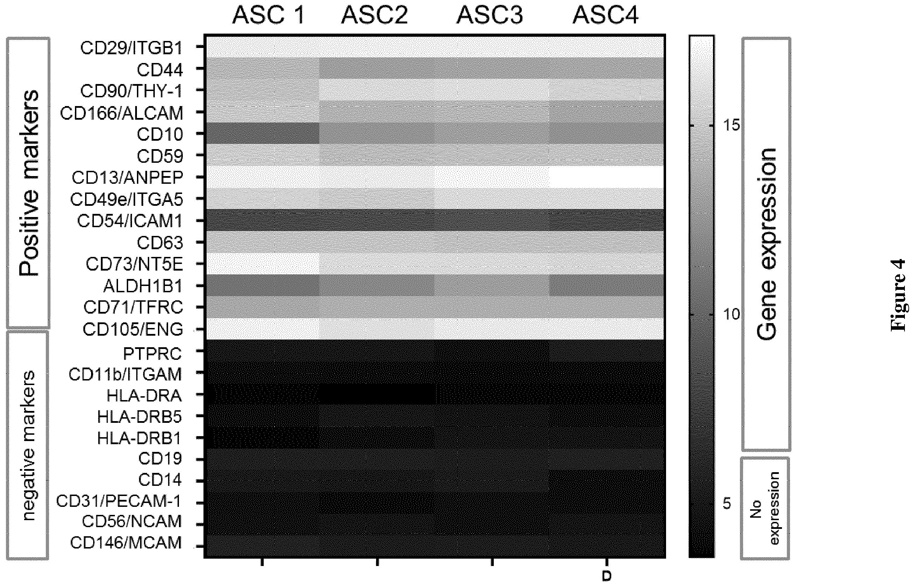

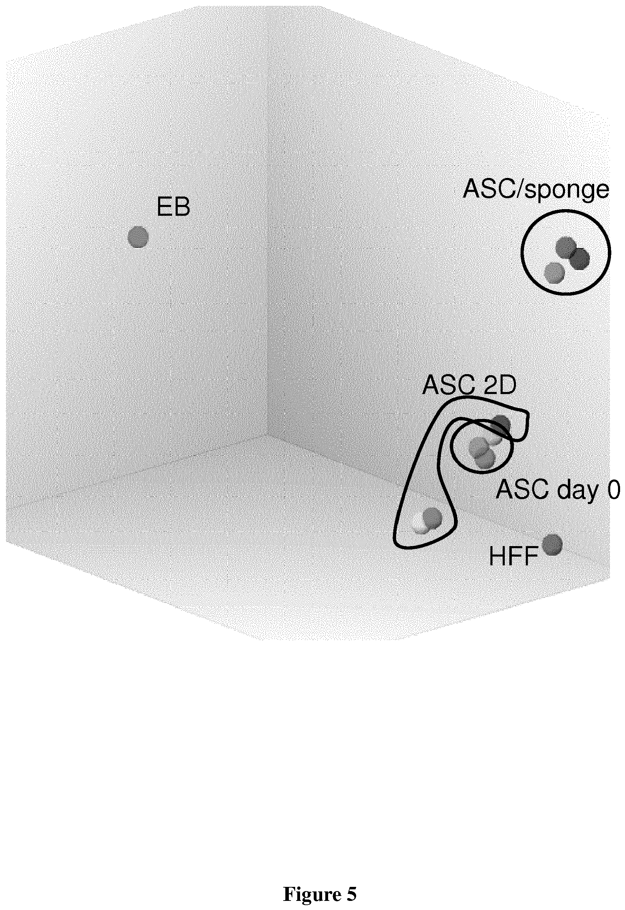

The cells in both culture conditions retained their ASC identity, as highlighted by the transcription of 14 genes reported to be expressed by ASC and the lack of ten molecules known to be absent in ASC (**Figure 4**). A Principal Component Analysis (PCA) was performed to compare the global gene expression profile of ASC at day 0, ASC/2D at day 8 and ASC/sponge at day 8 (**Figure 5**). PCA is a statistical procedure used for identification of a smaller number of uncorrelated variables known as principal components from a larger set of data. The technique is widely used to emphasize variation and capture strong patterns in a data set. To evaluate a possible fibroblastic differentiation in the sponge, human foreskin fibroblast (HFF) were also included in the analysis as control cultured as described below. As a "non-regenerative" control, 2 weeks-old embryoid bodies (EB) derived from the human embryonic stem cell line HS420 were also included.

### Fibroblast preparation and culture

Human foreskin fibroblasts (ATCC CCD-1112SK) were cultured in DMEM culture medium (Life Technologies) supplemented with 10% fetal calf serum (Gibco) and 1% penicillin-streptomycin (Gibco).

### Embryoid bodies preparation and culture

The HS420 embryonic stem cell line (gift from Dr Outi Hovatta, Karolinska Institute, Sweden) was cultured on Matrigel (Corning) - coated tissue culture flasks in Nutristem medium (Biological Industries) until 50% of confluency. 10 uM Rock inhibitor Y27632 was added in the Nutristem culture medium. After 24h, cells were detached with Accutase (Thermofisher) and aggregated into spheroids of 1000 cells by using Aggrewell-400 (STEMCELL technologies). 24h after the aggregation, spheroids were cultured for 10 days in suspension under constant agitation in differentiation medium (KnockOut DMEM (Thermofisher), 15% KnockOut serum replacement (Thermofisher), 2 mM L-glutamine (Sigma-Aldrich), 1% Penicillin/Streptomycin solution (Life Technologies), 1% of MEM Non-Essential Amino Acids Solution (Life Technologies), 0.1 mM β-mercaptoethanol (Sigma Aldrich)).

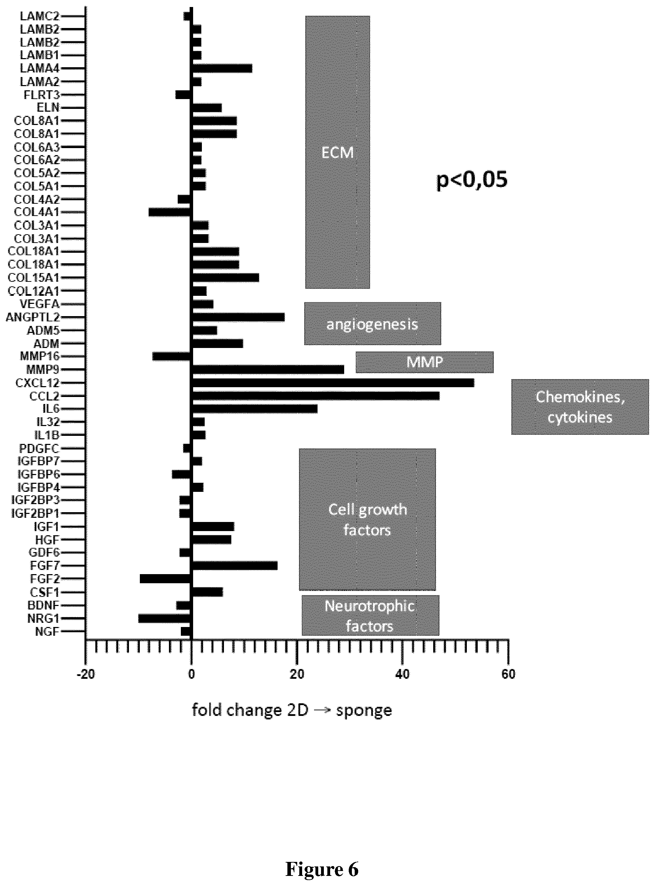

ASC/2D after 8 days of culture showed a slightly different gene signature than ASC/2D at day 0, with the exception of one line. Furthermore, ASC/sponge cells formed a distinct cluster in the PCA analysis than ASC/2D cells cultured for the same time (8 days), indicating that the sponge culture condition induces sizable and specific gene-transcription modification. ASC cultured in either the sponge or in 2D conditions did not differentiate in fibroblaste-like cells or embryoid bodies. Two hundred-fifteen mRNA corresponding to the functional families involved in the wound healing process were thus compared in these latter conditions, including: (i) ExtraCellular Matrix (ECM) proteins; (ii) angiogenic factors; (iii) matrix remodeling proteases; (iv) inflammatory factors (cytokines, chemokines); (v) cell growth factors and (vi) neurotrophic factors involved in wound healing. Among the screened mRNA, 75 were found to be expressed in both 2D and sponge conditions (value higher than the cut off of 6) and 48 showed a significant (p<0.05) regulation between the two conditions (**Figure 6**). In the majority of cases (34 out of 48 genes), the sponge culture conditions resulted in an increased gene expression as compared to the 2D condition. These included several ECM proteins such as collagens and laminins; angiogenic factors such as ANGPTL-2, VEGF and adrenomedullins (ADM) and the Matrix MetalloProtease MMP-9, which is strongly involved in the wound healing process. The quantitatively most upregulated genes corresponded to CXCL-12, CCL-2, IL-6 and FGF-7. CXCL-12 (SDF-1) is a strongly chemotactic agent for lymphocytes and mesenchymal stem cells during inflammation (Baumann et al., 1987, J Biol Chem, 262(20), 9756-68). It also plays an important role in angiogenesis by recruiting endothelial progenitor cells from the bone marrow (Zheng et al., 2007, J Cardiovasc Pharmacol, 50(3), 274-80). CCL-2 (MCP-1) and IL-6 are factors attracting inflammatory cells after tissue injury (Carr et al., 1994, Proc Natl Acad Sci USA, 91(9): p. 3652-6*;* Xu et al., 1996, J Leukoc Biol, 60(3), 365-71*).* FGF-7 (or Keratinocyte Growth Factor, KGF) is a potent growth factor for epidermis growth/formation. A minority of genes was instead down regulated, including COL4A1, MMP16, FGF2 and NRG1. The extent of downregulation was however small and never attending a fold change higher than 10.

Together, these observations suggest that ASC cultured according to the method of the invention acquire an enhanced healing potential as compared to standard culture, particularly by upregulating genes involved in angiogenesis, inflammation and matrix remodeling. Interestingly, the strong up-regulation of FGF-7 supports the possibility to grow an epidermal layer over the ASC/sponge composite.

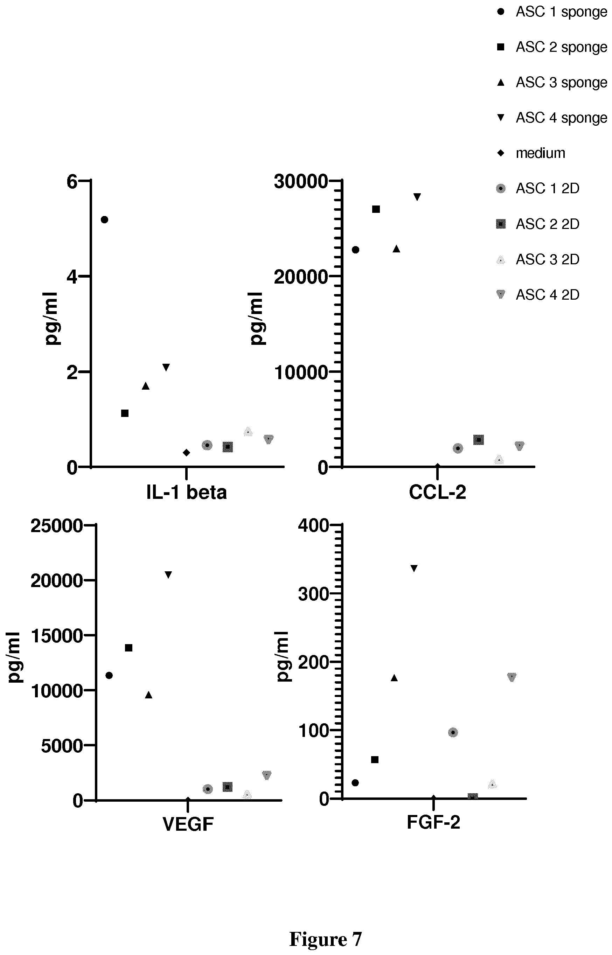

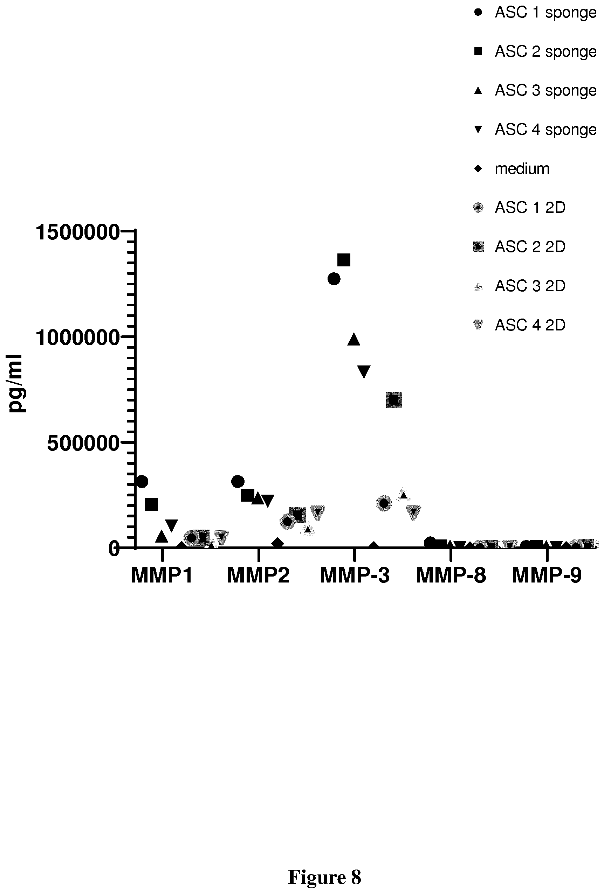

The expression of selected genes, important for the wound healing, was confirmed at protein level by measuring their concentration in culture supernatants. ASC cultured in 2D were once again compared with the same ASC cultured in the sponge condition. ASC lines from 4 different patients were analyzed (**Figure 7**). 5.10⁶ ASC were maintained in standard culture as a monolayer on plastic (2D) in 15 ml of medium or introduced within a sponge culture on 1ml of medium. After one week, factors were measured in supernatants. The concentration of IL-1β, FGF-2, CCL-2 and VEGF was strongly increased in the supernatants from sponge conditions (**Figure 7**). A similar observation was done with MMP1 and MMP3 (**Figure 8**). Of note, the expression level of the above-mentioned molecules was far higher (10'000-100'000x) than the concentration effect due by the culture condition. These data confirmed that the sponge condition, by maintaining ASC in a compact tissue-like structure, is capable of increasing the local concentration of healing factors, a property important for our approach.

### Example 3: Assessment of the long-term regenerative properties of the ASC/sponge composite

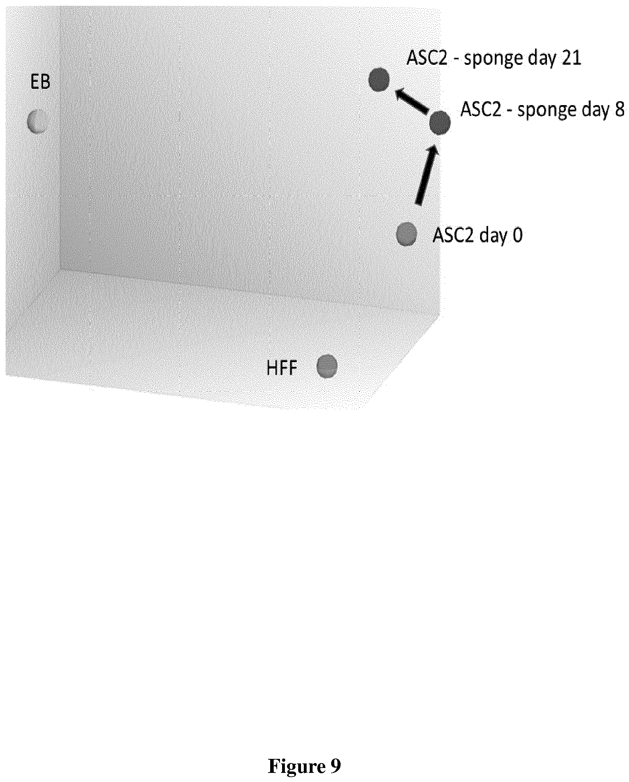

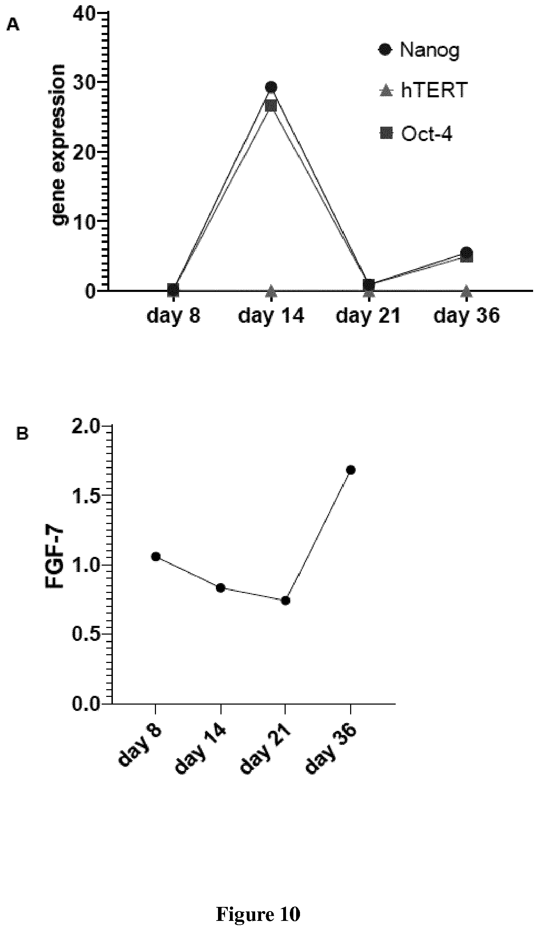

The stability of ASC and their long-term regenerative properties when cultured according to a method of the invention was assessed since, based on clinical observations, a long and continuous release of healing factors (approximately one month) is expected to be necessary for an effective healing process. At the mRNA level, a PCA performed with one ASC line (ASC 2) cultured according to a method of the invention showed that the global gene expression profile evolves along 3 weeks of culture (**Figure 9**). Of note, the ASC line remains distinct from HFF, excluding fibroblastic differentiation, as well as from embryoid bodies. ASC are a heterogeneous population of stromal cells including subtypes with proliferating/ sternness properties expressing Nanog and Oct-4. A significant increase of these sternness markers after 2 weeks in sponge conditions was noticed by Q-RT-PCR analysis (**Figure 10**), followed by down-regulation until day 36. A similar kinetic was observed with ASC cultured as spheroids (unpublished observations), suggesting that tissue-condition first favor proliferation of cells before stabilization. A first round of asymmetric divisions of stem cells, or positive selection of stem cells by the environmental conditions could precede evolution towards a more mature tissue. Importantly, for clinical cell therapy application, the ASCs cultured according to a method of the invention do not induce the tumorigenicity-associated marker hTERT (**Figure 10**).

### Example 4: Tissue substitute material protected by a reconstructed epidermis

Epidermis, the cellular layer covering the dermis in the skin, functions as a physical and mechanical barrier to the external environment. Upon stimulation by a pathogen trigger, keratinocytes in the epidermis also produce a large amount of peptide with anti-microbial properties and contribute to the establishment of the inflammatory response aimed at efficiently preventing microbial invasion in the host. During the later stage of wound healing, keratinocytes migrate from the edge of the wound over the newly formed granulation tissue to firmly close the wound and re-establish the lost protective barrier. In chronic wounds, the epidermis does not regenerate correctly, and in case of large wound, keratinocyte even fail to reconstitute a closed barrier. Therefore, it was tested as follows whether it would be possible to reconstruct an epidermis over the ASC / sponge material of the invention (**Figure 1B****, step f2)**).

The obtained ASC/sponge materials of the invention cultured for 8 days as obtained under step e) of Example 1 from two independent ischemic donors were covered with keratinocytes (7) prepared from the same patients as described below.

At day 8, the sponge/ASC composite **(3)** was removed from the hemipermeable membrane **(4)** and placed into a top chamber **(18)** (= 24-well insert with a hemi permeable membrane at the bottom) of a cell insert (**8**) (ThinCerts PET-membrane, 3,0 µm pore, 0.6x10⁶ cm² density) (Greiner) floating over 1 ml of ASC culture medium (**9**). The insertion of the sponge/ASC composite into a cell insert for the culture with keratinocytes is required to physically constrain the ASC/Sponge composite and allows the proper stratification of keratinocytes on one single side of the composite. The sponge/ASC was then covered, in the top chamber, with 500 µl of fully-supplemented CnT-Prime Epithelial Culture Medium (CELLnTEC) containing 200'000 autologous keratinocytes. Upon 24h, the remaining medium contained in the top chamber was replaced by an equal volume of 3D differentiation medium (CELLnTEC). The day after, the insert was moved to a deep-well plate containing 4 ml of ASC media (the hemi permeable membrane **(4)** at bottom of insert (**8**) floating on this media (**9**)), and the media covering the cellular sponge in the top chamber was removed. The epidermis was then allowed to stratify above the sponge/ASC at the air interface **(10)** for 10 to 15 days to allow the formation of a stratified epidermis **(11).** The media in the bottom chamber was replaced every three days. Upon application on a skin wound (as defined below), the ASC/sponge/epidermis can be covered by a protective layer of polyurethane **(12).**

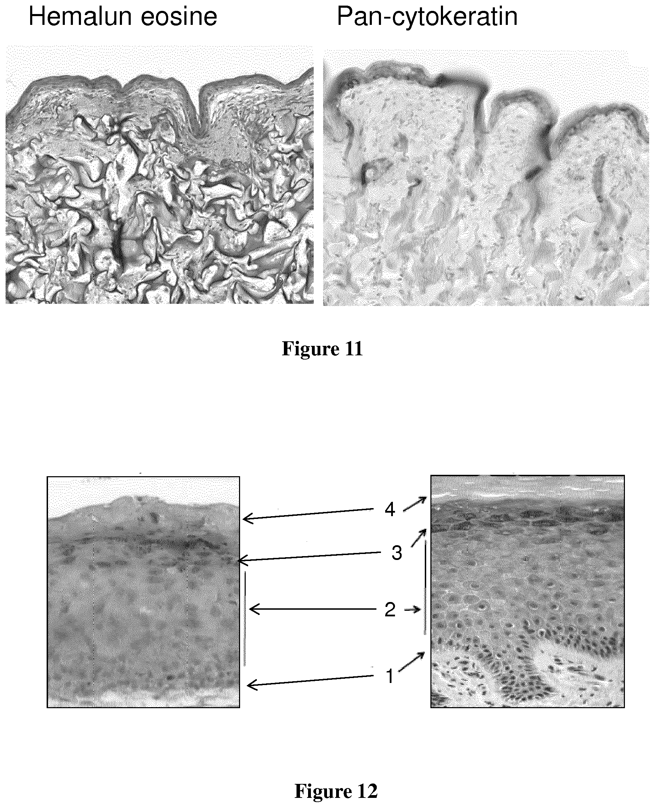

Upon 10 days of culture at the air-liquid interface, an epidermal sheet (**11**) was observed by H&E staining and confirmed by staining with a pan-keratin antibody (**Figure 11**). Of note, the epidermis failed to develop over empty sponges, used as controls. Similarly, direct culture of keratinocytes over PET-transwell membrane (standard culture condition for epidermis reconstitution in vitro) in ASC-medium resulted in the arrest of keratinocyte growth, while the same cells could develop an epidermal-like sheet in the presence of appropriate keratinocyte-media. This data strongly indicate that ASC-media does not support keratinocyte growth and that the presence of ASC in the sponge is critical for the effective development of an epidermal layer, most likely due to the production of needed factors for keratinocytes. Consistent with this hypothesis, it was noticed that ASC produce high transcript levels of an important keratinocyte survival and growth factors, FGF7 (also called Keratinocyte Growth Factor, KGF) when cultured in the sponge condition (**Figure 6**). ASC in the sponge maintained and even up-regulated the ability to produce FGF-7 for over one month *in vitro* as assessed by Q-RT-PCR (**Figure 10B**).

Although forming a continuous layer already at day 10 of culture, the epidermis was not completely stratified, as observed morphologically. The culture period was thus extended to 20 days in order to verify whether the ASC/sponge composite of the invention could allow the formation of a completely stratified epidermis. This was indeed the case as revealed by H&D staining and illustrated in **Figure 12** and schematized on **Figure 1B** (14) as ASC/sponge/keratinocytes skin substituted composite.

### Example 5: Characterization of the tissue substitute material of the invention of Example 4

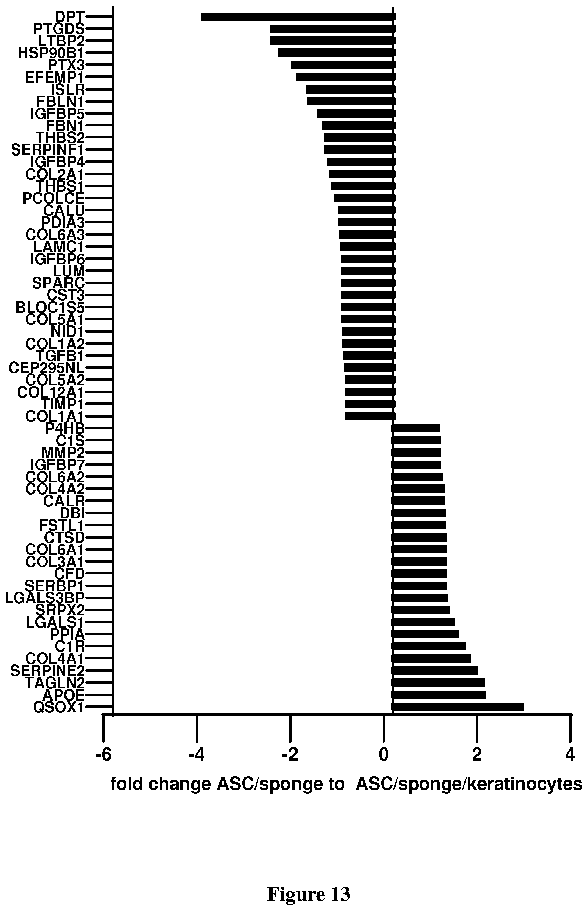

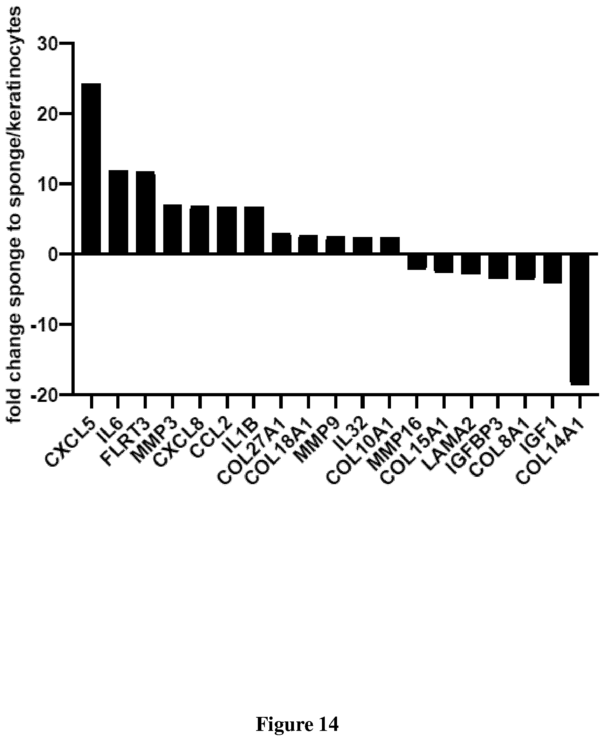

The influence of the epidermis layer on the regenerative properties of the ASC/sponge of the invention was characterized by comparing the full gene expression profile of 3 ASC/sponge of the invention (prepared as described in Example 1) versus 2 ASC/sponge/keratinocytes skin substituted composite of the invention (prepared as described in Example 4) generated from the same donors (ASC2, ASC3 and ASC4) after three weeks (one week of ASC expansion in the sponge, followed by two weeks of epidermis generation). The experiment was performed in biological triplicate to allow statistical analysis (three different ASC lines from three ischemic patients: ASC2, ASC3, ASC4). The 75 genes involved in regeneration and expressed by ASC/sponges (see previous data, **Figure 6**) were compared and ordered to track the most significant changes (**Figure 13**). Overall, the addition of the epidermis did not interfere, rather ameliorated, the healing properties of the ASC/sponge. Twenty genes showed a significant modulation (p<0.05) (**Figure 14**). Among them, 11 genes were very mildly affected (5 up-regulated and 6 downregulated, fold change < 3). Only one gene, collagen 14A1, showed a marked downregulation while 7 genes were strongly up-regulated by the presence of the epidermal layer.

Of note, these latter included several factors involved in wound healing, generally indicating that the ASC/sponge/epidermis composite is at least as efficient as the ASC/sponge composite to produce healing factors. The experiment design did allow distinguishing whether the gene transcription modification observed are due to an enhanced production of healing factors by the ASC or to an additional contribution from keratinocytes.

The impact of epidermis on the healing properties of the ASC/sponge composite was also investigated by screening the expression of the 68 ASC-most conserved and abundant secreted proteins, as outlined in the literature (Kapur et al., 2013, Biochimie, 95(12), 2222-8). Among them, 58 were found to be expressed with a signal higher than the cut off. Only dermatopontin (DPT), a component of ECM, was significantly down-regulated (p<0.05) by epidermis introduction (data not shown). Overall, these data indicate that presence of keratinocyte does not significantly alter the ASC identity and the associated secretome.

Altogether, those data show that it is possible to re-generate an epidermal layer on top of the ASC/sponge composite which would guarantee a faster and more efficient wound healing while simultaneously helping in maintaining the wound "sterile" by avoiding infections.

## Claims

1. A method for the preparation of a tissue substitute material, said method comprising the following steps:
a) Providing Adipose-derived Stem Cells (ASCs);
b) Loading the said ASCs into a scaffold sponge;
c) Placing the obtained loaded scaffold sponge onto to a hemi-permeable membrane floating over an ASC culture medium;
d) Incubating under ASC culture conditions the obtained floating loaded scaffold sponge to let the ASC growing and expanding within the said floating scaffold sponge;
e) Removing the cultured floating loaded scaffold sponge from the hemi-permeable membrane and obtaining an ASC-containing sponge suitable as a tissue substitute material.

2. A method according to claim 1 further comprising a step f2) wherein the ASC-containing sponge obtained under step e) is further coated with keratinocytes in keratinocyte culture medium to allow the formation of a stratified epidermis over the surface of the covered ASC-containing sponge.

3. A method according to claim 1 further comprising the following steps:
f2) placing the obtained ASC-containing sponge in a top chamber of a cell culture insert containing a hemipermeable membrane at the bottom, said cell culture insert floating on the surface of a culture medium and covering the ASC-containing sponge with keratinocytes in keratinocyte culture medium to form a keratinocyte-covered ASC-containing sponge within the said cell culture insert;
**g2)** incubating the keratinocyte-covered ASC-containing sponge under keratinocyte culture conditions thereby defining air interface conditions over the keratinocyte layer and allowing a stratified epidermis to grow on the surface of the ASC-containing sponge under said air interface conditions;
**h2)** removing the keratinocyte-covered ASC-containing sponge from the top chamber of the cell culture insert and obtaining an ASC-containing sponge covered with a stratified epidermis as a tissue substitute material.

4. A method according to any on of claims 1 to 3 wherein the ASC-containing sponge obtained in the last step is further coated with a protective membrane such as a vapour-permeable membrane (e.g. a polyurethane film).

5. A method according to any one of claims 1 to 4, wherein the scaffold sponge is loaded with about 100'000 to 200'000 autologous or heterologous Adipose-derived Stem Cells.

6. A method according to any one of claims 1 to 5, wherein the hemi-permeable membrane is made of polytetrafluoroethylene, polycarbonate, polyethylene terephthalate or cellulose esters.

7. A method according to any one of claims 1 to 6, wherein the floating loaded sponge is incubated under step d) for from about 5 to 20 days under ASC culture conditions.

8. A tissue substitute material comprising a scaffold sponge comprising ASCs expanded within said scaffold sponge under air-liquid interface conditions.

9. A tissue substitute material according to claim 8 wherein the scaffold sponge is a gelatin or collagen or collagen-gelatin sponge.

10. A tissue substitute material according to claim 8 or 9 wherein the tissue substitute material comprises about 100'000 to 1'500'000 ASCs.

11. A tissue substitute material according to any one of claims 8 to 10, further comprising a protective membrane such as keratinocyte layer or a vapour-protective membrane (e.g. a polyurethane film).

12. A tissue substitute material obtainable from a method according to any one of claims 1 to 7.

13. A tissue substitute material according to any one of claims 8 to 12 for use in tissue regeneration or tissue engineering or for the treatment of internal organ and skin wounds, in particular chronic ulcers.

14. A wound dressing, a reconstruction tissue a cosmetic composition or a soft tissue filler comprising a tissue substitute material according to any one of claims 8 to 12.

15. A kit for wound dressing comprising at least one tissue substitute material according to any one of claims 8 to 12.
